# EUROPEAN PATENT APPLICATION

(11) **EP 3 888 719 A1**
(43) Date of publication of application: **06.10.2021**
(21) Application number: 20000230.1
(22) Date of filing: 29.06.2020
(51) Int. Cl.: A61L 31/10, A61L 31/14, A61L 31/16

(54) **PATIENT CUSTOM MADE TRABECULAR OSTEOSYNTHESIS PLATE WITH ANTIBIOTIC COATING**

(30) Priority: 30.03.2020 CO 20003883
(71) Applicant: Techfit Digital Surgery Inc., Daytona Beach, Florida 32114 (US)
(72) Inventor: Martínez Carett, Juan Pablo, La Estrella (CO)
(74) Representative: Maldonado Jordan, Julia

(57) **Abstract**

The present invention relates to an osteosynthesis plate for fixation or treatment of bone fractures or injuries, which is patient custom made which anatomically adjusts and has an antibiotic bone cement coating for the treatment of infection in a focused and agile manner, thus reducing side effects.

## Description

### Technical field

The present invention relates to the field of biomedical engineering, particularly it relates to an implant for fixation or treatment of fractures or bone injuries, specifically formed by an osteosynthesis plate intended to improve the bone adjustment or fixation, while helping to treat infections or reducing a risk thereof, providing antibiotics in a focused, and minimally invasive manner, and reducing secondary effects or risks, wherein said plate anatomically adjusts to the patient tissue and has an antibiotic bone cement coating.

### Background of the invention

Osteosynthesis plates are used for fixation or treatment of fractures or bone injuries. For complex reconstruction cases, the patient custom made plates allow a better consolidation and a less surgical time, using the computer assisted surgical planning through the use of 3D images and anatomic models.

In addition, some patients present cases of infection which must be treated so the body can accept the implants. For the treatment of these infections, the patent can be subjected to an antibiotic treatment, however, by being systemic and not focused, the time and dosage must be much greater causing in some cases undesired side effects and postponing the time for surgery.

On the other hand, bone cement with antibiotic has been used in orthopedics for a more focused treatment of infection, however, applications so far used are for solutions in skull or for cases in which a bone graft or spacer can be used, since cement as such does not support the same load as a nail or osteosynthesis plate. Cemented intramedullary nails are also used for controlling infections and bone fixation, however, for certain fracture lines, plates above nails are used, and the plates can be useful for mor superficial infections on the bone.

In addition, a standard coated material affects its original geometry, does not guarantee a complete integration of the base material and the coating, and can feature detachments.

In this regard, there is a plurality of disclosures in the state of the art related to implants or osteosynthesis plates, with different geometries and coatings, within which is document CN204562348, which teaches a novel medicine carrying apparatus with reticular shape. This reticular shape includes a reticulate body, screw holes, medicine carrying holes, medicine carrying grooves. The medicine carrying holes are blind. The medicine distribution in the grooves is provided in a side of the reticulate body, both the grooves and the hole have antibiotics and promote bone recovery.

However, the invention defined in this anteriority features the disadvantage of not being patient custom made for treating osteosynthesis, while not mentioning an antibiotic bone cement coating.

On the other hand, document CN201542731 can also be found, which teaches an antibacterial bone plate. A plurality of through holes are uniformly arranged on a bone plate body and filled with antibiotic bone cement. As the bone plate body is provided with the through holes, the antibiotic bone cement is positioned by aid of said through holes when connected with the bone plate avoiding displacement of the antibiotic bone cement.

However, the invention defined in this anteriority has the disadvantage of not being patient custom made for treating osteosynthesis, while not having a trabecular geometry, nor having perforations for fixation to the bone via screws.

In the state of the art, document KR101846836B1 can also be found, which features an osteosynthesis plate for periosteum injury prevention which includes a metal fixing plate (12), and a plurality of screw holes (14) formed on the fixing plate, wherein fixing screws (15) pass through the screw holes (14) while closely attaching the fixing plate (12) to the bones around the fracture site, so as to fix the bones around the fracture site. The fixing plate (12) is provided with a slit-shaped discharge hole (16) for connecting the screw holes (14) adjacent to each other, and bleeding and fluid discharged from the periosteum are discharged through the discharge hole (16).

However, this invention features the disadvantage that the plate is not patient custom made, nor features the use of antibiotic bone cement or any other kind of coating.

On the other hand, document US20130171410A1 discloses a flexible body comprising a polymer film having a first surface and an opposing second surface. The polymer film has a plurality of apertures extending from the first surface to the second surface; in addition, it comprises a plurality of raised lips protruding from the first surface such that each of the plurality of apertures is surrounded by a series of raised lips. In addition, it discloses a method of producing a polymer film comprising the steps of: placing a polymer solution into a one sided mold having a plurality of protrusions extending from a bottom of the mold wherein the polymer solution is characterized by a viscosity that inhibits the unaided flow of the polymer throughout the mold; urging the polymer solution around each of the plurality of protrusions; and solidifying the polymer solution.

However, this invention features the disadvantage of being a flexible but not solid material, which does not behave as a coating, nor entirely adjusts in a trabecular geometry, whereby it would modify the dimensions of the final assembly.

Finally, document KR1020150104948 consists in a bone fixing plate having screw holes for inserting a screw fastened to a bone. The bone fixing plate includes: an auxiliary fixing layer composed of a biodegradable organic polymer material for having the bottom surface in contact with the bone by being configured in a form to cover a fracture area of the bone and the surrounding area thereof; and a main fixing layer composed of a biodegradable organic polymer material for having a suitable shape for connection in the fracture area by being coupled to the auxiliary fixing layer to partially cover the top of the auxiliary fixing layer.

However, the present document does not teach or disclose a plate with trabecular geometry which adapts to the patient geometry, while featuring the disadvantage of not including antibiotic elements, while the coating does not adjust to the plate.

According to the above, it is clear that there is a need in the state of the art to design a patient custom made osteosynthesis plate, which has an antibiotic bone cement polymeric coating, which is introduced in the plate porous geometry, guaranteeing its dimensions and avoiding detachments.

In addition, it is necessary that said plates have high-resistance, low weight, low profile, and biocompatible properties, and avoid contaminants in the implant preparation process.

### Brief description of the drawings

The present invention can be more clearly understood from the following figures where the components and results associated to the present element are shown, as well as the novel characteristics relative to the state of the art, wherein the figures do not intend to limit the scope of the invention, which is only provided by the appended claims, where:

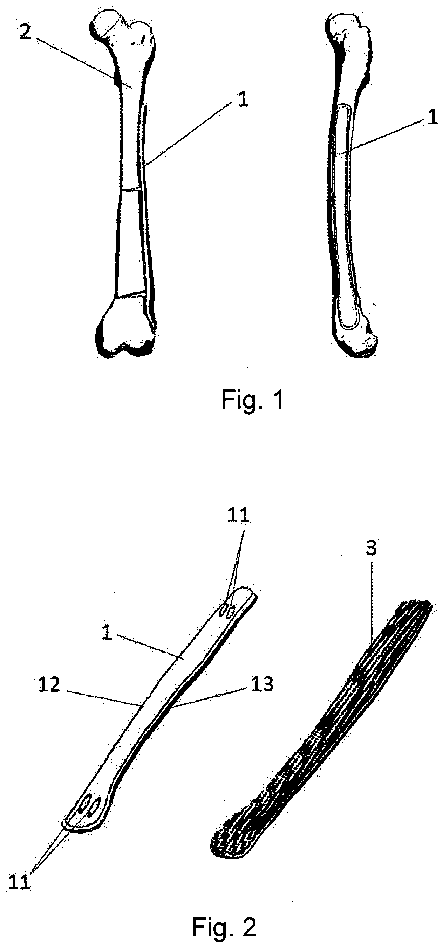

Figure 1 corresponds to front and side views of a fractured bone, fixed with the osteosynthesis plate.
Figure 2 corresponds to a perspective view of the osteosynthesis plate, wherein each of the elements forming such plate are shown.

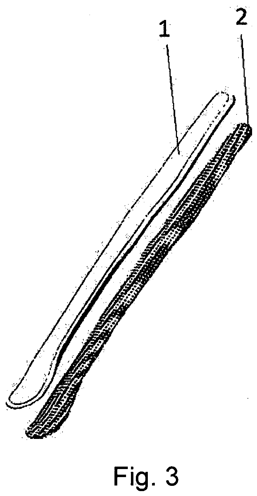

Figure 3 corresponds to a perspective view of the most general configuration of the osteosynthesis plate.

### Detailed description of the invention

The present invention is directed to an osteosynthesis plate for fixation or treatment of bone fractures or injuries, which is composed by the following components:
An osteosynthesis plate (1) for treating a bone (2), the plate being formed by a top surface (12) and a bottom surface (13), and a series of fixation through holes (11), wherein said plate (1) is trabecular or porous, wherein said bottom surface (13) is in the bone (2) direction, wherein said bottom surface (13) is coated with antibiotic releasing polymeric material, in order to help both with consolidation of osteotomy and treating the infection. Wherein said plate adjusts to the patient anatomy by being patient custom made with CAD/CAM techniques and computer assisted surgical planning through the use of 3D images and anatomic models.

In addition, the volume of said osteosynthesis plate is trabecular or porous between 40% and 60%, wherein said polymeric coating is introduced through said trabeculae or pore space, in order to keep the plate original dimensions, avoiding undesired thickness increase, or modifications in the shape of the patient custom made plate, while the through holes (11) free to passing fixation screws, which are planned in advance, such that it is not necessary to pierce the coating in surgery, which may cause cracks or damages in said coating.

In this regard, the antibiotic releasing polymeric coating is bone cement with an antibiotic percentage between 1% and 1.25%, which, by being located on the trabeculae, avoids undesired detachments thereof, achieving to thus comply its recovery function, while avoiding cement contamination, during formation of the implant.

Said osteosynthesis plate is made in a biocompatible material such as surgical metals or high-performance engineering polymers, wherein said material has a low thickness or gauge, low weight and high resistance.

In a preferred embodiment, said plate is manufactured in surgical steel or titanium, has a volume raging between 40% and 60% weight of the trabecular or porous osteosynthesis material, and is coated with antibiotic bone cement.

Although the above description defines the preferred embodiments of the present invention, said invention is not limited thereto, but, instead, it also intends to include in the scope of the application all the variations or modifications which are evident for those skilled in the art and which do not affect and modify the spirit of the invention.

## Claims

1. Osteosynthesis plate (1) for bone (2) treatment, comprising a top surface (12) and a bottom surface (13), and a plurality of fixation through holes (11), wherein said plate (1) is trabecular or porous, wherein said bottom surface (13) is in the bone (2) direction, wherein said bottom surface (13) is coated with antibiotic releasing polymeric material.

2. The osteosynthesis plate according to claim 1, **characterized in that** its volume is trabecular or porous between 40% and 60%.

3. The osteosynthesis plate according to any preceding claim, **characterized in that** the polymeric coating is introduced in the trabeculae of the plate (1), keeping its initial dimensions and geometry, leaving the fixation through holes (11) free.

4. The osteosynthesis plate according to any preceding claim, **characterized in that** the antibiotic releasing polymeric coating is bone cement with an antibiotic percentage between 1% and 1.25%.

5. The osteosynthesis plate according to any preceding claim, **characterized in that** its geometry adjusts to the patient anatomy.

6. The osteosynthesis plate according to any preceding claim, **characterized in that** it is patient custom made with CAD/CAM techniques and computer assisted surgical planning.

7. The osteosynthesis plate according to any preceding claim, **characterized in that** it has a low profile according to the anatomic zone where it is located, whether it is lower or upper limb.

8. The osteosynthesis plate according to any preceding claim, **characterized in that** its manufacturing material is metal or engineering polymers.

9. The osteosynthesis plate according to any preceding claim, **characterized in that** it is fixed to the bone (2) through screws.
